# EUROPEAN PATENT APPLICATION

(11) **EP 1 449 505 A1**
(43) Date of publication of application: **25.08.2004**
(21) Application number: 03003284.1
(22) Date of filing: 24.02.2003
(51) Int. Cl.: A61F 13/58

(54) **Elastic fastening tab, fastening system and method for manufacturing the same**

(71) Applicant: 3M Innovative Properties Company, St. Paul, Minnesota 55133-3427 (US)
(72) Inventor: Löscher, Claus, 41468 Neuss (DE)
(74) Representative: VOSSIUS & PARTNER

(57) **Abstract**

An elastic laminate fastening tab (2) comprising adjacent first and second closure elements (6, 8), each having a first face (10, 12) and a second face (14, 16), wherein the first face of each closure element is coated with a pressure sensitive adhesive (20, 22), an elastic film 18 attached to each closure element first face, the elastic film having two end portions (24, 26) connected to the first and second closure elements, respectively, and an intermediate portion therebetween, and a blanking film (28, 30) interposed between the closure elements and the elastic film. The invention also provides a fastening system comprising such a fastening tab and a method of manufacturing the same.

## Description

### Field of the Invention

The present invention relates to a novel elastic laminate fastening tab and a fastening system for use in disposable absorbent articles, particularly adult incontinence articles, and to a method of manufacturing the same.

### Background of the Invention

Disposable absorbent articles often use tab fasteners that are provided with elastic to allow for adjustment in the fit and a more secure and snug fit. For example, US-A-3,800,796 (Jacob) teaches disposable diapers with elastic tabs wherein the tabs have an elastic segment between two inelastic segments. One of the inelastic segments is used to attach the tab to the diaper and the other inelastic segment has a pressure sensitive adhesive (PSA) to allow the tab to close the diaper. The tab can be made such that the entire tab is formed of an elastic material and the two terminal segments are rendered inelastic, for example by laminating an inelastic material to the end segments. In US-A-3,800,796, the elastic would be exposed to the adhesive if a tab precursor laminate were directly wound into a roll. This is problematic in that elastics generally can become permanently bonded to or contaminated by adhesives. This construction does not protect the elastic from direct contact with the adhesive. Further, the tabs vary in thickness which may inhibit the formation of a stable roll of the precursor tab fastener laminate.

US-A-4,522,853 (Szonn) also discloses an elastic tab adhesive closure for disposable diapers. The tabs have an intermediate (middle) elastic piece and adhesive coated end portions, and the elastic is not protected from direct contact with adhesive layers. The constructions are complicated and have multiple different layers that would result in considerable variation in the tab thickness making stable roll formation difficult.

US-A-4,643,729 (Laplanche) also discloses a complex elastic fastener for disposable diapers that comprises three parts, i.e., a first lateral side part (inelastic, for attaching to the diaper during manufacturing), a second lateral side part (inelastic, containing PSA for closing the diaper), and an elastic central part. The two lateral parts are parts of a single piece composed of a support band provided with a transverse pre-cut line, which are separated at the time of use (along the pre-cut line). The two lateral parts are bonded to the central elastic part with adhesive bands and the lateral parts also have adhesive on the face opposite the elastic part for bonding to the diaper (one end for manufacturers bond and the other end for closing the diaper in use). The elastic tabs taught in US-A-4,643,729 again would not protect its elastic material from adhesive contact when wound in a roll.

US-A-4,778,701 (Pape) discloses a roll of laminated strips from which separate elastic closure tabs can be cut. This is a pre-laminated elastic tab closure with a central elastic portion and inelastic end portions, one for attaching to the diaper during manufacturing and one for the consumer to use to close the diaper. The elastic is also not protected from exposure to the adhesive without an extra piece of protective material. The thickness of this material would also vary significantly over the width of the tab which is undesirable.

US-A-4,795,456 (Borgers) discloses an extensible tab for disposable diapers that is stabilized (inextensible) until the user deploys the tab for diapering. This inextensibility of the elastic central segment is achieved by the tab construction including a non-extensible web (e.g. a release tape or liner) that bridges the inextensible end segments (as does the elastic segment) until the tab is opened for use. The elastic is again not protected from the adhesive. Also, the construction requires multiple adhesive layers and film layers such that it would be costly and difficult to manufacture.

US-A-4,834,820 (Kondo) discloses an elastic diaper closure tab containing an elastic sheet and an inelastic retaining sheet which is directly bonded to the elastic sheet in the terminal end regions and the inelastic sheet has a cut-off groove in the center portion. When using the diaper the consumer manually rips apart the retaining sheet along the cut-off groove, whereupon the center of the elastic sheet is made flexible (elastic) as the retaining sheet no longer bridges the elastic sheet between the areas where the retaining sheet and the elastic sheet are bonded. The tab further has adhesive on the underside of the elastic sheet to anchor the tab to the side of the diaper (manufacturers bond) and to be used for closing the diaper. The structures (elastic tabs) again require multiple layers of adhesive and fail to protect the elastic from direct contact with the adhesive layers if wound into a roll.

US-A-5,057,097 (Gesp) discloses elastic diaper tape tabs wherein there is adhesive on the end segments and a central segment free of adhesive, and wherein the tape backing is a multilayer elastic film (e.g. a co-extruded elastic with inelastic skin layers). The elastic is protected from direct exposure to the adhesive layers but the terminal portions are not necessarily inelastic as the entire tab is generally elastic which could result in the tab failing during use. Also, the elastic is always exposed prior to use.

JP-A-60-215803 (Hida) discloses a tab for disposable diapers having non-extensible end regions and an extensible sheet that connects the end regions and in which at least one part of the overlapping area of the end members and the extensible sheet is non-bonded. Again, the elastic in this construction is not protected from contact with the adhesive if wound into a roll form and a complicated multiple step construction process is required.

EP-A-1 000 598 (Selen et al.) discloses a fastening system comprising a mechanical connection tape for the fastening of two articles or two parts of an article to each other. This fastening system comprises a mechanical connection tape provided with a fastening tape having a first section adapted to be fixed to a first article or a first part of an article and having a second section provided with a first fastener member of a two-part mechanical fastener. The mechanical connection tape comprises a target tape having a first surface adapted to be fixed on a second article or a second part of the article and having a second surface provided with a second fastener member of said two-part mechanical fastener. The first and second members are capable of being mechanically engaged with and mechanically disengaged from each other. The second surface of the target tape is provided with a first end section within which the first member cannot be engaged with the second member thereof. The first end section of the second surface of the target tape is distal from the first section of the fastening tape when the first and second members are mechanically engaged. This fastening system is generally known as a 3-tape-fastening sytem.

US-A-6,463,633 (Sangani et al.) discloses a stretchable tape having a multilayer construction including an adhesive layer for tape mounting of non-extensible and extensible layers. This fastening tape includes a first terminal segment or outer end portion, a central segment and a second terminal segment or outer end portion in its lengthwise direction. In its thickness direction, the tape includes a non-extensible facestock layer, an adhesive layer, adhesive-inhibiting masking layers, and an extensible layer. A separation line or plane is located within the central segment. The separation line is formed by slits and connecting ties. The slits extend in a width direction in respect to the tape and through the thickness of the layer, and the slits are intermittently interrupted by maintenance of the non-extensible facestock layer to form the ties. The adhesive layer includes a surface secured to the surface of the non-extensible facestock layer and a remote surface. The portion of the remote surface adjacent the first terminal segment may be used to mount the fastening tape to a release tape. The portion of the remote surface adjacent the second terminal segment may be used to close a diaper about an infant or wearer by securing it to a front diaper panel. The adhesive inhibiting masking layer is provided along the surface of the adhesive layer within the central segment of the tape adjacent the separation line. The masking layer extends across the entire width of the tape and along at least a portion of the length of the tape within the central segment of the tape. Portions of the adhesive layer on each side of the masking layer respectively secure the elastic layer to the non-extensible layer adjacent the first and second terminal segments of the tape. The adhesive inhibiting masking layer is formed by printing using an ink which may be thinned with a solvent. The thickness of the masking layer is insignificant in that it does not exceed about 3% of the combined thickness of the non-extensible facestock layer, adhesive layer and extensible layer. The printed ink masking layer may have a thickness of about 4 µm (microns).

EP-A-0 832 630 (Bräunig et al.) relates to a diaper closure tape comprising a first inelastic carrier portion attachable to a diaper backside, a second inelastic carrier portion releasably attachable to a diaper frontside, and an elastic intermediate portion between the two carriers portions. The two carriers have an open surface in their connecting areas with the intermediate portion, and the intermediate portion consists of a thermoplastic elastic material which penetrates in a softened state into the surface openings of the carrier portions, thus mechanically anchoring the intermediate portion in the carrier portions.

### Summary of the Invention

It is an object of the present invention to provide an improved elastic fastening tab which is comfortable to the wearer and can be manufactured easily and precisely. It is a further object of the invention to provide an improved fastening system which is particularly useful for adult incontinence diapers.

The elastic laminate closure or fastening tab of the present invention comprises adjacent first and second closure elements, each having a first face and a second face, wherein the first face of each closure element is at least partially coated with a pressure sensitive adhesive. The tab further comprises an elastic film having two end portions which are attached to the first face of each of the first and second closure elements, and an intermediate portion therebetween. A blanking film is attached to the first face of each of the first and second closure elements and is interposed between the closure elements and the elastic film such that the elastic film is not in contact with the adhesive coating of the closure elements at its intermediate portion.

The first and second closure elements are preferably initially provided as a fastening tape which is centrally or intermittently slit. The blanking film preferably also comprises two separate portions provided by centrally slitting the blanking film, said two separate portions being attached to the first and second closure elements, respectively. The slit trough the fastening tape and/or the blanking film is typically a straight slit but a wavy slit or differently shaped slit may also be applied.

After applying the fastening tab of the invention to two articles to be connected, for example to the front waist portion and back waist portion of a diaper, the fastening tab can be stretched and can provide both elasticity and flexibility to the closure.

In accordance with another aspect, the present invention provides a fastening system in the form of a 3-tape-laminate comprising an elastic laminate fastening tab as described above, a target tape having a first surface adapted to be fixed on an article, like a diaper, and a second surface adapted to receive a fastening portion of said fastening tab, and a release tape onto which said first surface of the target tape may be bonded.

The present invention furthermore relates to methods for manufacturing such an elastic laminate fastening tab and fastening system.

A particular advantage of the fastening tab of the present invention vis-à-vis prior art fastening tabs is that it can be manufactured reliably, precisely and at low costs. In particular, it is much easier to provide an additional blanking film for partially covering the adhesive layers of the closure elements than, e.g., in-line ink coating a very thin layer onto the adhesive. Use of a blanking film allows a lamination process to be used and avoids a strip coating process. Similar advantages are achieved over applying an adhesive coating only on parts of the inelastic closure elements as in the prior art.

A further advantage of the fastening tab of the present invention is that due to the present construction of the blanking film and closure elements any pull forces are applied immediately on the elastic element, while in accordance with prior art constructions connecting portions must be broken by the first pull force before activating the elastic properties. In the fastening tab of the present invention no such stabilizing connecting portions are necessary, but the closure elements including their adhesive coating and the blanking film are preferably substantially completely cut and only connected by the elastic film.

The elastic and stretchable fastening tab and system of the present invention improves diaper fit and can substitute a conventional elastic waist band. The fastening system of the present invention allows an increased thickness of the fastening tape finger lift, thus improving the grasping by the hand of a user. The materials of the fastening system of the present invention can be selected such that they are printable. The elastic film used in the present invention may be initially hidden and become visible when the fastening tab is being stretched.

The elastic fastening tape and fastening system of the present invention can be manufactured in endless form and converted into a stable roll. Both planetary and level wound roll formats as well as festooning may be used as roll format.

### Brief Description of the Drawings

- Fig. 1: is a perspective view of a disposable absorbent article using the elastic fastening tab of the present invention.
- Fig.2: is a schematic side view of the fastening tab of the present invention.
- Fig. 3: is a perspective side view of the fastening tab illustrated in Fig. 2.
- Fig. 4: is a schematic side view of a first embodiment of a 3-tape-laminate fastening system of the present invention.
- Fig. 5: is a schematic side view of another embodiment of the 3-tape-laminate fastening system of the present invention without target tape.
- Fig. 6: is a side view of the 3-tape-laminate fastening system shown in Fig. 5 with target tape.
- Fig. 7: is a schematic side view of an alternative embodiment of the fastening tab of the invention.
- Fig. 8: shows a method for manufacturing the fastening tab and fastening system of the invention.
- Fig. 9-11: demonstrate, in schematic exploded views, the various positions of the fastening system of the invention during its use.

### Detailed Description of Preferred Embodiments

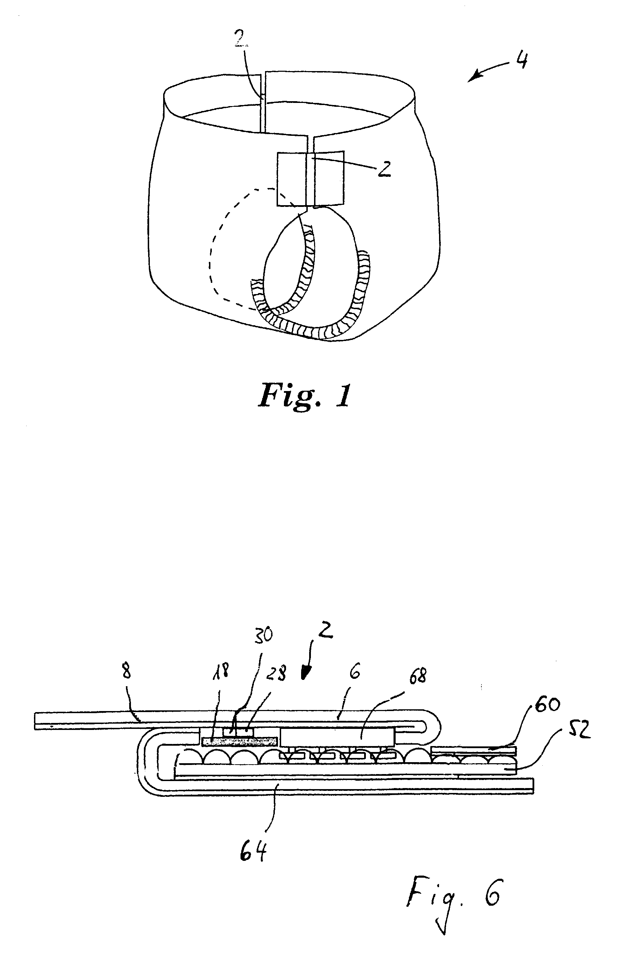

Fig. 1 shows an elastic fastening tab 2 of the present invention in use on a disposable absorbent article, i.e., diaper 4. The elastic fastening tab 2 is extended and attached at two opposing side regions of the disposable absorbent article 4.

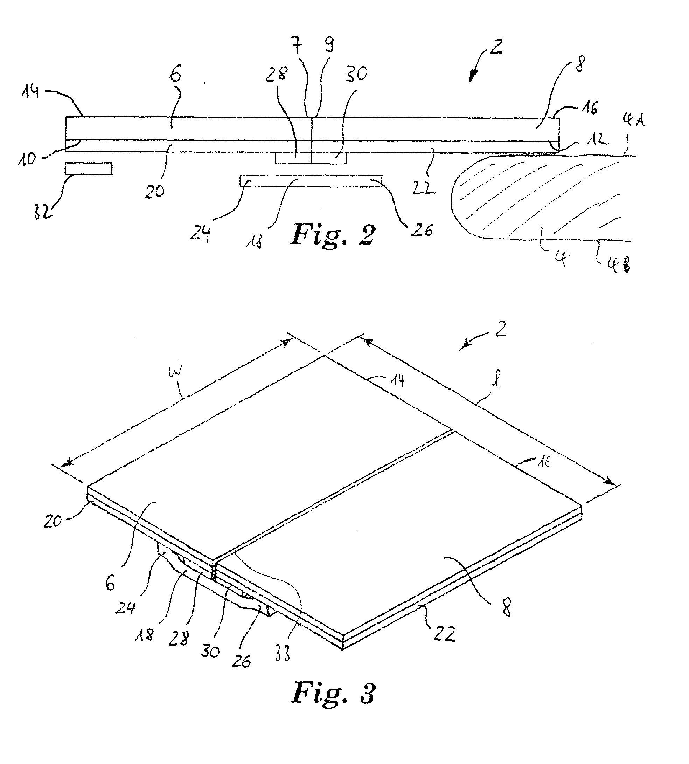

As shown in Figs. 2 and 3, the elastic laminate fastening tab 2 of the present invention comprises two adjacent first 6 and second closure elements 8 having opposing terminal ends 7, 9. The closure elements 6, 8 are preferably made of a nonwoven material, or a laminate of a nonwoven and a film layer, or a monolayer film, like a polyolefine film. Each of the closure elements 6, 8 has a first face 10, 12 and a second face 14, 16.

An elastic film 18 is shown unattached in Fig. 2 but is in the final tab attached with its two end portions 24, 26 to each closure element first face 10, 12. The elastic film may be a monolayer film but preferably is in the form of a coextruded film. An elastic film covered on one or both sides with polyolefine, polyamide or polyester nonwoven or an elastic nonwoven may also be used. Preferably the elastic film 18 is attached approximately symmetrically with respect to the two opposing terminal ends 7, 9 of the adjacent elements 6 and 8 but optionally the elastic film may also be attached asymmetrically. The elastic film 18 can be made from any suitable material and is preferably made from materials as disclosed in WO 99/19388.

The first face 10, 12 of both closure elements 6, 8 is preferably completely coated with a pressure sensitive adhesive 20, 22. The elastic film 18 has its first end portion 24 and its second end portion 26 adhesively connected to the first and second closure elements 6 and 8, respectively. The elastic film 18 is attached to the closure elements by way of adhesive 20, 22. Supplementary bonding means such as a thermal bond, an ultrasonic bond or cold pressure bonding, can be used in addition to the adhesive bond. An intermediate portion of the elastic element 18 between the end portions 24, 26 is inhibited from being adhesively connected to the closure elements 6, 8 by means of an interposed blanking film comprising two portions 28, 30 provided on the closure elements 6, 8 at or near their terminal ends. In the embodiment shown in Figs. 2-6 the overall length of the blanking film 28, 30 is smaller than the length of the elastic film 18 so as to allow direct attachment of the end portions 24, 26 of the elastic film 18 to the closure elements 6, 8. The blanking film preferably is a separate film that is laminated to the closure elements 6, 8 during manufacturing of the laminate fastening tab 2. The blanking film is preferably made of a transparent material but can also be colored or made of a nonwoven with an optional laminated film. The length of the blanking film 28, 30, or the distance between the attachment sites of the elastic film 18 to the two closure elements 6, 8 determine the width of the active elastic zone or absolute elongation.

At an outer end of one of the closure elements 6, 8 a finger lift 32 is provided which is shown unattached in Fig. 2 and laminated during the manufacturing process.

The disposable absorbent article, i.e., diaper 4, is schematically shown in Fig. 2. The first closure element 6 is used to close the diaper after its application and is commonly called "user's end". The second closure element 8 is used to attach the fastening tab at the diaper back sheet 4A and is usually called "manufacturer's end". The diaper top sheet at the inside of the diaper is shown at 4B.

The various layers are all of such a thickness that the thickness variation across the fastening tab 2 is preferably less than about 50%, and more preferably less than about 40%. More precisely, the thickness of the closure elements 6, 8 is preferably in the range of between 80 to 120 µm, more preferably about 95 to 105 µm. The thickness of the adhesive 20, 22 is typically in a range of between 30 and 40 µm, preferably about 35 µm. Moreover, the thickness of the interposed blanking film 28, 30 is typically 8 to 20 µm, preferably 10 to 14 µm, and most preferably about 12 µm. The elastic film 18 preferably has a thickness in the range of between 150 and 250 µm, preferably 180 to 220 µm and most preferably about 200 µm.

The width w of fastening tab 2 (see Fig. 3) may vary depending on the use of the fastening tab and is generally in the range of between 10 to 70 mm, preferably between 20 and 50 mm.

In the direction of their length (see Figs. 2 and 3) the various elements of the fastening tab of the present invention may have the following dimensions: the two closure elements 6, 8 measured together may have a length of about 50 to 100 mm, preferably 60 to 80 mm and most preferred about 70 mm. The two portions 28, 30 of the blanking film together may have a length of about 4 to 10 mm, preferably 5 to 8 mm and most preferred about 6 mm, wherein preferably both portions have the same length. The elastic film 18 may have a total length of about 10 to 20 mm, preferably about 12 to 18 mm and most preferred about 15 mm, and is preferably applied centrally over the line separating the two closure elements 6 and 8 and their opposing terminal ends 7, 9. The finger lift element 32 may have a length of about 5 to 15 mm, preferably 8 to 12 mm and most preferred about 10 mm.

The two adjacent closure elements 6, 8 at their opposing terminal ends 7, 9 are preferably in an abutting face to face relation such that they cover substantially the whole elastic film 18. Thus, the elastic film 18 is covered prior to use and is protected from adhesive contamination while also minimizing thickness variation across the fastener tab 2 in the central region of the tab. There are also functional and aesthetic advantages. The elastic film 18 can be colored differently and the closure elements 6, 8 may be provided with designs so as to provide distinguishable contrasts. When the user extends the tab 2 by a certain distance the previously covered elastic film 18 is then clearly exposed, in particular when the blanking film 28, 30 is transparent. This provides a clear visual indication of the extent to which the elastic film 18 is stretched which is also a direct indication of the force being applied by the elastic film. When two opposing tabs 2 are used on a diaper 4, as shown in Fig. 1, the level of force could then be easily adjusted so that each tab 2 is extended to the same degree. Generally, one can easily see that the elastic film 18 is being used and adjust the degree of stretch as appropriate.

Similarly, the opposing ends of adhesive layers 20, 22 and blanking films 28, 30 are in a face to face relation such that they cover substantially all of the elastic film 18 prior to use.

Typically, the two closure elements 6, 8 of the elastic fastening tab 2 of the present invention are manufactured from a single continuous web material that is completely covered by an adhesive layer. During the manufacturing process a film ultimately forming the blanking film is then centrally applied onto the adhesive coating of the web. The web material, the adhesive layer and the film are then centrally slit or cut to form the two separate closure elements 6, 8 each covered with adhesive layers 20 and 22, respectively, and the separate blanking films 28, 30 of approximately equal lengths. The cut line 33 is shown in Fig. 3. Subsequently, the elastic film 18 in the form of a continuous tape or sheet is adhesively connected at its lateral end portions 24, 26 to the respective adhesive layers 20, 22. The blanking film is thus completely covered and prevents an adhesive connection between the intermediate portion of the elastic film 18 and the closure elements 6, 8. The continuous roll of fastening tab tape can then be cut into individual fastening tabs as illustrated in Fig. 3. These fastening tabs can be stretched due to the elastic film 18. Upon stretching, the gap between closure elements 6 and 8 widens, thus providing elasticity and flexibility to the closure.

The closure elements 6, 8 are preferably a laminate of a nonwoven and a film layer but can also be a single film, preferably a polyolefine film, like a polypropylene film or polyethylene film, or a nonwoven without a film layer. The nonwoven provides softness while the film layer provides a barrier for the adhesive and/or dimensional stability. The nonwoven preferably is a spunbond nonwoven, having a basis weight from 20 g/m² to 50 g/m², wherein the film layer is extrusion laminated to the spunbond nonwoven. The film layer is preferably adjacent the pressure-sensitive adhesive layer 20, 22. The film layer can also be separately provided and adhesive laminated, sonic bonded or otherwise conventionally attached to the nonwoven layer. The pressure-sensitive adhesive layers 20, 22 are provided on the closure elements 6, 8 to both attach the elastic film 18 to the closure elements 6, 8 and further to attach the closure elements 6, 8 to the disposable garment 4, or other article on which it may be used, or to other elements. Preferably one and more preferably both closure elements 6, 8 have an area beyond the elastic film 18 where the pressure-sensitive adhesive layers 20, 22 are exposed and that can be used to attach the closure elements 6, 8 to the disposable garment 4 or alternatively could be used to attach other elements such as mechanical fastener elements.

The elastic film end portions 24, 26 may have at least one zone that is inelastic to provide for more secure adhesion to the closure elements 6, 8. This inelastic zone extends generally at least 0.5 mm, preferably at least 1 mm to 10 mm and may in a preferred embodiment extend over the entire area of the end portions 24, 26 attached to the adhesive layers 20, 22. This provides for secure adhesion of the elastic film 18 to the adhesive layers without excessive use of the elastic material. The intermediate elastic region of the elastic film 18 generally is of a length of from 5 to 20 mm, preferably from 10 to 15 mm for most uses in a disposable absorbent article. The size of the elastic region depends on the size of the article being used with the tab 2 and the exact nature of the article, its intended adjustability and the elastic forces required. The elastic region could be formed of at least one elastic zone, which could extend at least 0.5 mm, preferably at least 1 to 5 mm or more. The elastic zones, if not continuous, would be separated by inelastic zones. The use of inelastic zones in the elastic region of elastic film 18 may be useful in adjusting the elastic properties of the elastic region.

The elastic film 18 is preferably a coextruded elastomeric laminate comprising at least one elastomeric core layer and at least one relatively nonelastomeric skin layer. When selected regions of the skin layer are stretched beyond its elastic limit and relaxed, the surface of the film becomes microstructured and the elastomeric core in this area is formed into an elastic region.

The elastomer forming the elastomeric core layer or, alternatively, the total elastic film 18 can broadly include any material which is capable of being formed into a thin film layer and exhibits elastomeric properties at ambient conditions. Elastomeric means that the material will substantially resume its original shape after being stretched. Further, preferably, the elastomer will sustain only small permanent set following deformation and relaxation which set is preferably less than 20 percent and more preferably less than 10 percent of the original length at moderate elongation. Generally, any elastomer is acceptable which is capable of being stretched to a degree that causes relatively consistent permanent deformation in a relatively inelastic skin layer. This can be as low as 20 % or 50% elongation. Preferably, however, the elastomer is capable of undergoing at least 100 % elongation. Elongations of up to 300 to 1200% and most preferably up to 600 to 800% at room temperature are also possible. The elastomer can be both a pure elastomer and blends with an elastomeric phase or content that will still exhibit substantial elastomeric properties at room temperature.

Preferred elastomers include block copolymers which are elastomeric such as those known to those skilled in the art as A-B or A-B-A block copolymers. These block copolymers are described, for example, in US-A-3,265,765; US-A-3,562,356; US-A-3,700,633; US-A-4,116,917 and US-A-4,156,673, the substance of which are incorporated herein by reference.

Styrene/isoprene, butadiene or ethylene-butylene/styrene (SIS, SBS or SEBS) block copolymers are particularly useful. Other useful elastomeric compositions can include elastomeric polyurethanes, ethylene copolymers such as ethylene vinyl acetates, ethylene/propylene copolymer elastomers or ethylene/propylene/diene terpolymer elastomers. Blends of these elastomers with each other or with modifying non-elastomers are also contemplated. For example, up to 50 weight percent, but preferably less than 30 weight percent, of polymers can be added as stiffening aids such as polyvinylstyrenes, polystyrenes such as poly(alpha-methyl)styrene, polyesters, epoxies, polyolefins, e.g., polyethylene or certain ethylene vinyl acetates, preferably those of higher molecular weight, or coumarone-indene resin. The ability to use these types of elastomers and blends provides the tab 2 of the invention with significant flexibility.

Viscosity reducing polymers and plasticizers can also be blended with the elastomers such as low molecular weight polyethylene and polypropylene polymers and copolymers, or tackifying resins such as Wingtack™, aliphatic hydrocarbon tackifiers available from Goodyear Chemical Company. Tackifiers can also be used to increase the adhesiveness of an elastomeric core layer to a skin layer. Examples of tackifiers include aliphatic or aromatic hydrocarbon liquid tackifiers, polyterpene resin tackifiers, and hydrogenated tackifying resins. Aliphatic hydrocarbon resins are preferred.

The above elastomers may also be used if the whole elastic film 18 consists of an elastomer and there is no inelastic skin layer.

The relatively inelastic skin layer is preferably formed of any semi-crystalline or amorphous polymer that is less elastic than the core layer(s) and will undergo permanent deformation at the stretch percentage that the elastomeric laminate will undergo. Therefore, slightly elastic compounds, such as some olefinic elastomers, e.g. ethylene-propylene elastomers or ethylene-propylene-diene terpolymer elastomers or ethylenic copolymers, e.g., ethylene vinyl acetate, can be used as skin layers, either alone or in blends. However, the skin layer is generally a polyolefin such as polyethylene, polypropylene, polybutylene or a polyethylene-polypropylene copolymer, but may also be wholly or partly polyamide such as nylon, polyester such as polyethylene terephthalate, polyvinylidene fluoride, polyacrylate such as poly(methyl methacrylate) and the like, and blends thereof. The skin layer material can be influenced by the type of elastomer selected. If the elastomeric core layer is in direct contact with the skin layer the skin layer should have sufficient adhesion to the elastomeric core layer such that it will not readily delaminate.

Additives useful in the skin layer include, but are not limited to, mineral oil extenders, antistatic agents, pigments, dyes, antiblocking agents, provided in amounts less than about 15%, starch and metal salts for degradability and stabilizers such as those described for the elastomeric core layer.

Other layers may be added between the core layer and the outer layers, such as tie layers to improve the bonding of the layers. Tie layers can be formed of, or compounded with maleic anhydride modified elastomers, ethyl vinyl acetates and olefins, polyacrylic imides, butyl acrylates, peroxides such as peroxypolymers, e.g., peroxyolefins, silanes, e.g., epoxysilanes, reactive polystyrenes, chlorinated polyethylene, acrylic acid modified polyolefins and ethyl vinyl acetates with acetate and anhydride functional groups and the like, which can also be used in blends or as compatiblizers in one or more of the skin or core layers. Tie layers are particularly useful when the bonding force between the skin and core is low. This is often the case with polyethylene skin as its low surface tension resists adhesion. However, any added layers must not significantly affect the microstructuring of the skin layer(s).

To provide for adjacent elastic and inelastic zones it is possible to either vary the skin layer or elastic layer in these regions or zones so that only certain areas are capable of being or becoming elastic. In an extreme case, the elastic core layer can be absent from a region or substantially thicker in a region to cause that region or zone to form an inelastic area such as described in US-A-5,057,097 (Gesp) or US-A-5,773,374 (Wood et al.), the substance of which are incorporated herein by reference. Alternatively, the elastic or skin layers can be treated in zones to weaken or strengthen them to provide elastic or inelastic zones, respectively. For example, zones or regions can be controlled to have lower overall modulus values that will preferentially yield before adjacent, in the direction of an orienting stress, higher modulus regions. This modulus control can be accomplished by a variety of methods that can involve the prelaminate formation stages, the formation stage, or post formation treatment of a particular laminate or laminate intermediate. Similarly, localization of stress, applied to the whole laminate, can result in preferential elongation in areas containing these localized stress regions. This stress control can also be effected by a variety of methods in any of a multitude of stages in the formation of the laminate. These strengthening or weakening treatments can include post formation annealing, selective crosslinking or selective plasticization, localized corona treatment, mechanical ablation, scoring, cutting out laminate material or indentation or the like, specific elastic zones can also be formed by controlled localized stretching as disclosed in US-A-5,344,691 (Hanschen), the substance of which is incorporated by reference.

After forming a zone activatable elastic laminate, the laminate is stretched past the elastic limit of the skin layer(s) exclusively or preferably in the lower modulus or preferred stress regions, which deform. The zone activated laminate then is recovered instantaneously, with time or by the application of heat. For heat activated recovery the inherent temperature of heat activation is determined by the materials used to form the elastic layer of the laminate in the first instance. However, for any particular laminate the activation temperature can be adjusted by varying the skin/core ratio of the laminate, adjusting the percent stretch or the overall laminate thickness. The activation temperature used for a heat shrink laminate is generally at least 80°F (26.7°C).

Activation will generally be accomplished by stretching the laminate in a direction substantially transverse to a primary extent of the film having spaced zones or regions of differing modulus or stress characteristics. At least one inelastic zone will preferably be in the end portions 24, 26 of the film 18 attached to the closure elements 6, 8. At least one elastic zone will be in the intermediate region of the film unattached to the closure elements 6, 8. Preferably inelastic zones will form at least 10 percent of end portion 24, 26, preferably at least 50 percent and in a preferred embodiment 100 percent. Preferably elastic zones will form at least 50 percent of the unattached intermediate region, preferably at least 80 percent and in a preferred embodiment 100 percent.

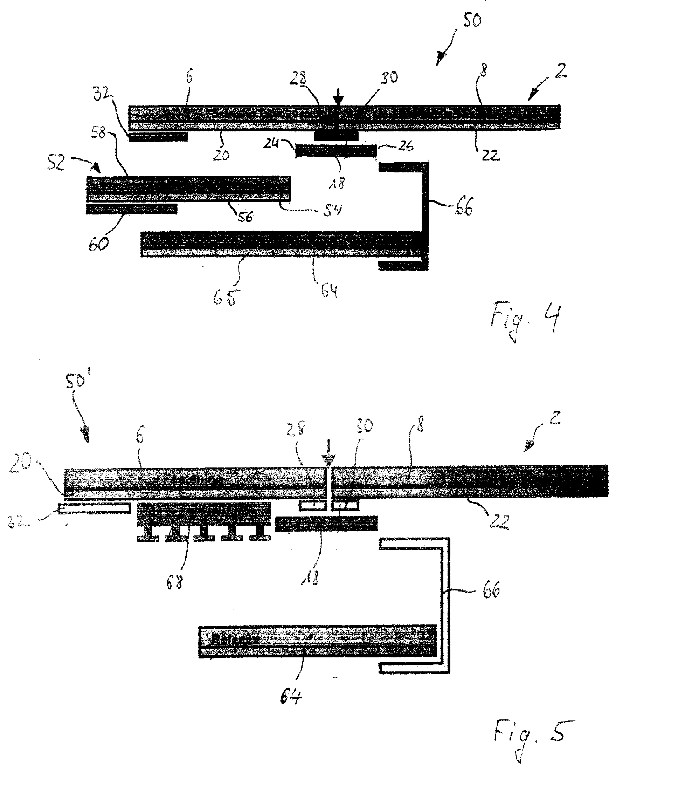

Fig. 4 illustrates a first embodiment of a 3-tape-laminate fastening system 50 in accordance with the present invention. This fastening system 50 is particularly useful for adult incontinence diapers. This fastening system comprises an elastic laminate fastening tab 2 as described above and shown in Figs. 2 and 3, a target tape 52 having a first surface 54 adapted to be fixed or selectively placed on an article, e.g., a disposable diaper, by means of a pressure sensitive adhesive 56, and a second surface 58 opposite the first surface 56 which is adapted to receive an adhesive layer 20 of the fastening tab 2 as fastening portion. The target tape 52 may be provided with a finger lift element 60 on the first surface 54. This finger lift element 60 is conveniently mounted to the target tape 52 by means of the adhesive 56. The target tape 52 is typically used in adult incontinence diapers to minimize the amount of target material on which the fastening tab 2 is to be applied. More precisely, the target tape 52 is applied to the expected closure position of the fastening tab 2 by means of the adhesive 56 during the first use of the diaper. Afterwards, the fastening tab 2 can be opened and reclosed by means of the finger lift element 32 at the end portion of the fastening tab 2 secured to the target tape 52 with the adhesive 20.

The 3-tape-laminate fastening system according to the present invention furthermore comprises a release tape 64 on which the target tape 52 may be secured by means of the adhesive 56. Furthermore, the fastening system is stabilized by means of a center stripe 66 connecting the release tape 64 and the fastening tab 2 and bonded to adhesive 65 on the back side of release layer 64. The release tape 64 may also be directly connected to the fastening tape 52 by folding its edge, thus creating an adhesive to adhesive bonding.

The dimensions of the fastening tab 2 of the fastening system 50 are essentially the same as described above in connection with Figs. 2 and 3. The dimensions of the other elements of the fastening system 50 are as follows. The target tape 52 preferably has a length of about 20 to 50 mm, preferably about 30 to 40 mm and most preferred about 35 mm. The finger lift 60 of the target tape 52 has a length of about 5 to 15 mm, preferably about 8 to 12 mm and most preferred about 10 mm. The release tape 64 has a length of about 30 to 70 mm, preferably about 40 to 60 and most preferred about 50 mm. The overlap between the release tape 64 and the finger lift element 60 of the target tape 52 is about 2 to 6 mm and preferably about 4 mm. The distance between the outer edges of the closure element 8 and the release tape 64 is preferably about 10 to 30 mm, more preferred about 15 to 25 mm and most preferred about 18 mm. The target tape 52 projects about 2 to 6 mm and preferably about 4 mm from the outer edge of the closure element 6. The distance between the elastic film 18 and the inner edge of the target tape 52 and the center stripe 66 is preferably less than 1 mm and most preferred about 0.5 mm.

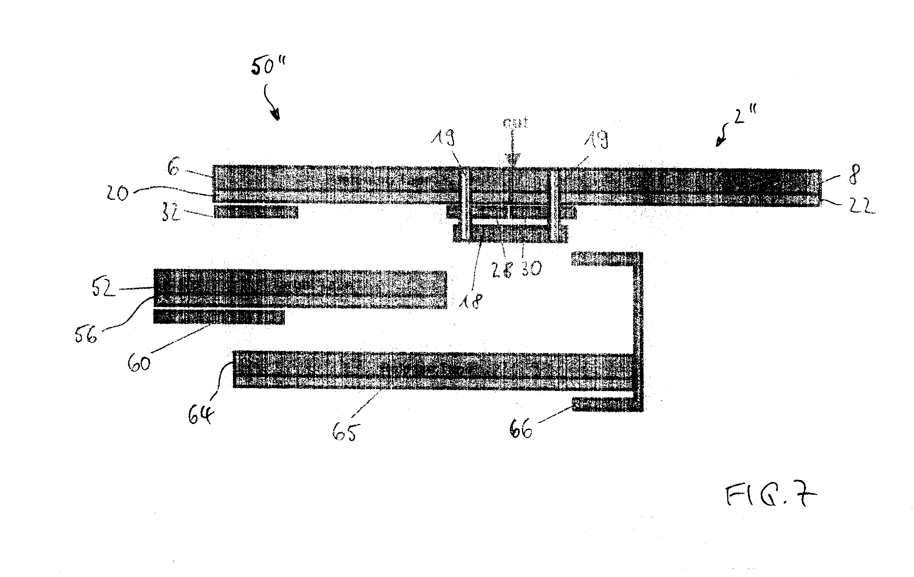

An alternative embodiment of the three-tape-laminate fastening system 50' of the present invention is illustrated in Fig. 5. In accordance with this embodiment a mechanical fastening system is employed. This mechanical fastening system comprises a first mechanical fastener component 68, e.g., hooks, adhered to the adhesive 20 provided at the fist closure element 6. Although not illustrated, in this embodiment the target tape comprises a second mechanical fastener component, e.g., a loop material or another mating hook component which functions as target zone for the closure. The mechanical fastener components can also be used in the fastening tab 2 of Figs. 2 and 3, if the target material is already provided on a diaper. This is schematically illustrated in Fig. 6. The integration of a hook and providing a loop tape as target tape results in an elastic 3-tape-mechanical fastener laminate which can be combined with frontal landing zones in order to provide a functional closure system.

Fig. 7 shows an alternative embodiment of a fastening tab 2" in a fastening system 50" of the present invention. In this embodiment the blanking film 28, 30 is extended so that its total length is larger than the length of the elastic film 18. In this embodiment the elastic film 18 can be fixed to the extended blanking film 28, 30 by means of thermobonding, ultrasonic welding or cold pressure bonding. The blanking film 28, 30 is preferably adhesively attached to the closure elements 6, 8 by means of the adhesive 20, 22, and additional bonding means, like thermobonding, ultrasonic bonding or cold pressure bonding may be used. Preferably the elastic film 18 and the two portions 28, 30 of the blanking film are attached to the closure elements 6, 8 in a single bonding step by means of thermobonding, ultrasonic bonding or cold pressure bonding, as indicated by reference signs 19, in Fig. 7. The width of the active elastic zone is essentially determined by the distance of the two bonding lines 19, 19 where the elastic film 18 is bonded to the closure elements 6, 8.

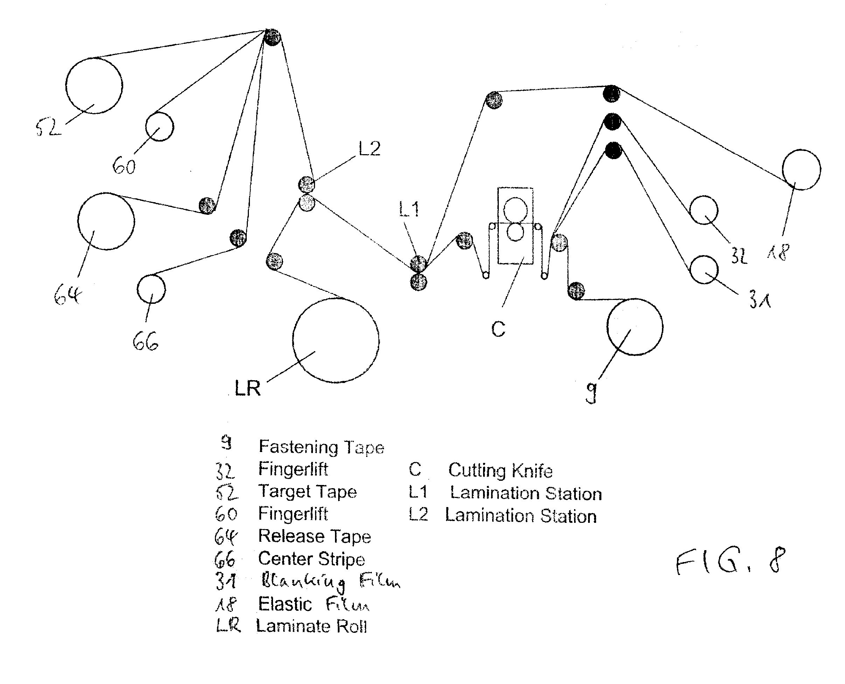

Fig. 8 shows a method for manufacturing the fastening tab and fastening system according to the present invention. Fig. 8 is a so-called single laminate roll process; twin-laminate roll processes are also feasible for the present invention.

In the method shown in Fig. 8 the material 9 ultimately forming the first and second closure elements 6, 8, the material 31 ultimately forming the two portions 28, 30 of the blanking film and the material 32 forming the finger lift of the fastening tab are provided in endless form from respective rolls via guiding rolls to a cutting section including cutting knife C where they are slit along their length and further to the first lamination station L1 where they are laminated to the elastic film 18 material also provided in roll form via guide rolls. As discussed above, lamination can be carried out by adhesive bonding but thermobonding, ultrasonic bonding or cold pressure bonding can be used supplementary or alternatively, in particular in the embodiment where the elastic film 18 is shorter than the blanking film 31. Alternatively, the materials 9 and 31 can also be cut to form closure elements 6, 8 and blanking film portions 28, 30 subsequently to being laminated to the elastic film 18. A partial cutting or providing a perforation line instead of fully cutting or slitting materials 9 and 31 is also an option.

The laminate including, still in the form of endless sheets, the closure elements 6, 8, the portions 28, 30 of the blanking film and the elastic film 18 is then provided to the second lamination station L2 as a first laminate. In the second lamination station L2 said first laminate is laminated to a second laminate including target tape 52 with finger lift 60, release tape 64 and center stripe 66 in the form of endless sheets. At the second lamination station L2 the fastening system according to the present invention is laminated and is then rolled up on laminate roll LR. Said fastening system in roll form can then be provided, e.g., to an in-line diaper production.

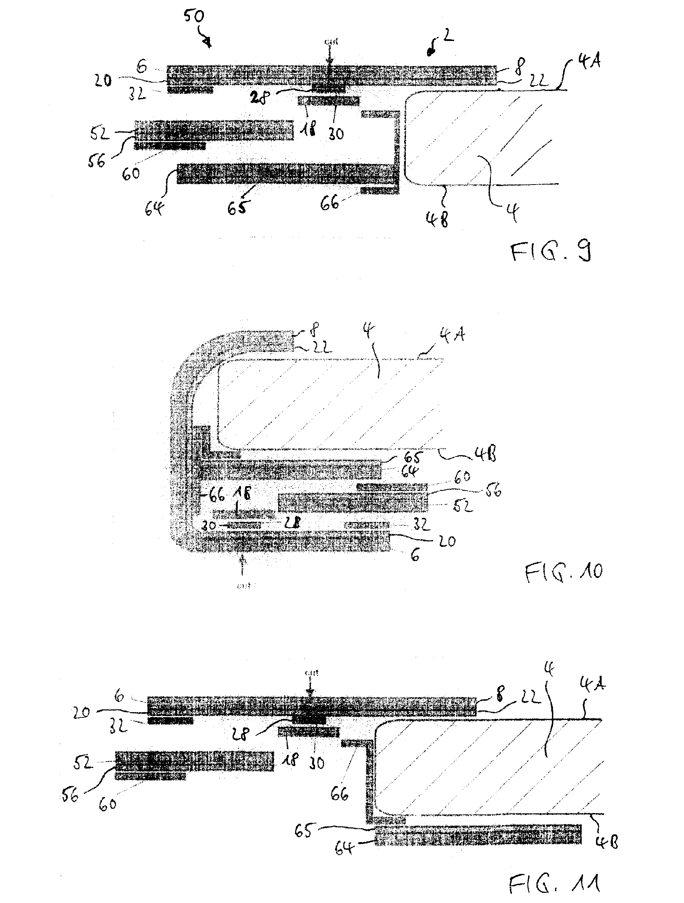

Figs. 9-11 show the positions of the fastening system of the present invention with respect to the disposable absorbent article, i.e., the diaper, during manufacturing and use. Fig. 9 is a view similar to Fig. 4 with diaper 4 including top sheet 4B at the inside of the diaper and diaper backside 4A attached to the manufacturer's end 8 of the fastening tab 2. In Fig. 10 the release tape 64, the target tape 52 and the user's end 6 of the fastening system are folded back onto the top sheet 4B at the diaper inside to provide a so called "Y-bond" by means of adhesive 65. This is the position in which the diaper including the fastening system according to the present invention is typically delivered by the manufacturer to the end user. At the first use of the diaper the user may then grab finger lift element 60 of target tape 52 to "unfold" the fastening system while the release tape 64 stays attached to the diaper top sheet 4B at the diaper backside. This position is schematically shown in Fig. 11. The user can then attach target tape 52 via its adhesive 56 at the diaper front side. Subsequently, the fastening tab 2 of the fastening system 50 can be opened and reclosed by the user by means of the finger lift 32 provided at the user's end 6 of the fastening tab 2.

## Claims

1. An elastic laminate fastening tab comprising adjacent first and second closure elements, each having a first face and a second face, wherein the first face of each closure element is at least partially coated with a pressure sensitive adhesive, an elastic film having two end portions attached to the first face of each of the first and second closure elements and an intermediate portion therebetween, and a blanking film attached to the first face of each of the first and second closure elements and interposed between the closure elements and the elastic film such that the elastic film is not in contact with the adhesive coating of the closure elements at its intermediate portion.

2. The elastic laminate fastening tab of claim 1 wherein the elastic film is attached to the closure elements by means of adhesive bonding, thermobonding, ultrasonic bonding and/or cold pressure bonding.

3. The elastic laminate fastening tab of claim 2 wherein the elastic film is attached to the closure elements by adhesive bonding and supplementary bonding by means of thermobonding, ultrasonic or cold pressure bonding.

4. The elastic laminate fastening tab of any of claims 1 to 3, wherein the end portions of the elastic film are at least partially inelastic.

5. The fastening tab of any of claims 1 to 4, wherein the blanking film comprises two separate portions being attached to the first and second closure elements, respectively.

6. The fastening tab of any of claims 1 to 5, wherein the total length of the blanking film is smaller than the length of the elastic film and the elastic film is directly attached to the first and second closure elements at its two end portions.

7. The elastic laminate fastening tab of any of claims 1 to 5, wherein the total length of the blanking film is larger than the length of the elastic film and the elastic film is attached to the first and second closure elements via the blanking film.

8. The fastening tab of any of claims 1 to 7, wherein the blanking film is transparent.

9. The fastening tab of any of claims 1 to 8, wherein the blanking film is a polyethylene, polyolefin or biaxially oriented polypropylene film, or a nonwoven with or without laminated film.

10. The fastening tab of any of claims 1 to 9, wherein the closure elements have a thickness in the range of between 80 to 120 µm, preferably 95 to 105 µm.

11. The fastening tab of any of claims 1 to 10, wherein thickness of the pressure sensitive adhesive is in the range of between 30 to 40 µm, preferably about 35 µm.

12. The fastening tab of any of claims 1 to 11, wherein the thickness of the elastic film is in the range of between 150 to 250 µm, preferably between 180 and 220 µm, and most preferably about 200 µm.

13. The fastening tab of any of claims 1 to 12, wherein the blanking film has a thickness of about 10 to 20 µm, preferably about 10 to 14 µm, and most preferably about 12 µm.

14. The fastening tab of any of claims 1 to 13, wherein the closure elements are made of a monolayer film, e.g., a polyolefin film, a laminate of a nonwoven and a film layer, or a nonwoven without film layer.

15. The fastening tab of claim 14, wherein the nonwoven is a spunbond nonwoven.

16. The fastening tab of claim 15, wherein the film layer is extrusion-laminated to the spunbond nonwoven.

17. The fastening tab of any of claims 1 to 16, wherein the pressure-sensitive adhesive is exposed on at least one closure element.

18. The fastening tab of any of claims 1 to 17, wherein at least one of the closure elements is provided with a mechanical fastening element.

19. The fastening tab of any of claims 1 to 18, further comprising a finger lift element on an outer edge of one of the closure elements.

20. A fastening system, particularly for adult incontinence diapers, comprising:
i) an elastic fastening tab of any of claims 1 to 19;
ii) a target tape having a first surface adapted to be fixed on an article and a second surface adapted to receive a fastening portion of said fastening tab; and
iii) a release tape.

21. The fastening system of claim 20, wherein said target tape is interposed between said fastening tab and said release tape.

22. The fastening system of claim 20 or 21, wherein the release tape is connected to the one of the closure elements by means of the target tape and to the other closure element by means of a center stripe.

23. The fastening system of any of claims 20 to 22, wherein the target tape comprises a finger lift element to enhance removability of the target tape from the release tape.

24. A method of manufacturing an elastic laminate fastening tab comprising the steps of:
i) providing a substantially inelastic web;
ii) coating one surface of the web with a pressure-sensitive adhesive;
iii) applying a blanking film onto the pressure-sensitive adhesive along the length of the web in a central portion of said web;
iv) slitting the web, the pressure-sensitive adhesive and the blanking film along their lengths;
v) attaching an elastic film to the two web portions on both sides of said blanking film to connect the two web portions, and
vi) cutting the thus formed laminate across its length to form said elastic laminate fastening tab.

25. A method of manufacturing a fastening system, particularly for adult incontinence diapers, said method comprising:
i) providing a substantially inelastic web;
ii) coating one surface of the web with a pressure-sensitive adhesive;
iii) applying a blanking film onto the pressure-sensitive adhesive along the length of the web in a central portion of said web;
iv) slitting the web, the pressure-sensitive adhesive and the blanking film along their lengths;
v) attaching an elastic film to the two web portions on both sides of said blanking film to connect the two web portions and to form a first laminate;
vi) providing a second laminate including a target tape, a release tape and a center stripe;
vii) laminating the first and second laminates together to provide a third laminate;
viii) rolling up said third laminate to form a laminate roll.

26. The method of claim 24 or 25, wherein the elastic film covers said blanking film completely and is directly attached to the two web portions.

27. The method of claim 24 or 25, wherein the blanking film is larger than the elastic film and the elastic film is attached to the two web portions via the blanking film.

28. The method of any of claims 24 to 27, wherein the elastic film is attached to the two web portions by means of adhesive bonding, thermobonding, ultrasonic bonding and/or cold pressure bonding.

## Amended claims

### Amended claims in accordance with Rule 86(2) EPC.

1. A fastening system, particularly for adult incontinence diapers, comprising:
i) an elastic laminate fastening tab comprising adjacent first and second closure elements, each having a first face and a second face, wherein the first face of each closure element is at least partially coated with a pressure sensitive adhesive, an elastic film having two end portions attached to the first face of each of the first and second closure elements and an intermediate portion therebetween, and a blanking film attached to the first face of each of the first and second closure elements and interposed between the closure elements and the elastic film such that the elastic film is not in contact with the adhesive coating of the closure elements at its intermediate portion;
ii) a target tape having a first surface adapted to be fixed on an article and a second surface adapted to receive a fastening portion of said fastening tab; and
iii) a release tape.

2. The fastening system of claim 1 wherein the elastic film is attached to the closure elements by means of adhesive bonding, thermobonding, ultrasonic bonding and/or cold pressure bonding.

3. The fastening system of claim 2 wherein the elastic film is attached to the closure elements by adhesive bonding and supplementary bonding by means of thermobonding, ultrasonic or cold pressure bonding.

4. The fastening system of any of claims 1 to 3, wherein the end portions of the elastic film are at least partially inelastic.

5. The fastening system of any of claims 1 to 4, wherein the blanking film comprises two separate portions being attached to the first and second closure elements, respectively.

6. The fastening system of any of claims 1 to 5, wherein the total length of the blanking film is smaller than the length of the elastic film and the elastic film is directly attached to the first and second closure elements at its two end portions.

7. The fastening system of any of claims 1 to 5, wherein the total length of the blanking film is larger than the length of the elastic film and the elastic film is attached to the first and second closure elements via the blanking film.

8. The fastening system of any of claims 1 to 7, wherein the blanking film is transparent.

9. The fastening system of any of claims 1 to 8, wherein the blanking film is a polyethylene, polyolefin or biaxially oriented polypropylene film, or a nonwoven with or without laminated film.

10. The fastening system of any of claims 1 to 9, wherein the closure elements have a thickness in the range of between 80 to 120 µm, preferably 95 to 105 µm.

11. The fastening system of any of claims 1 to 10, wherein thickness of the pressure sensitive adhesive is in the range of between 30 to 40 µm, preferably about 35 µm.

12. The fastening system of any of claims 1 to 11, wherein the thickness of the elastic film is in the range of between 150 to 250 µm, preferably between 180 and 220 µm, and most preferably about 200 µm.

13. The fastening system of any of claims 1 to 12, wherein the blanking film has a thickness of about 10 to 20 µm, preferably about 10 to 14 µm, and most preferably about 12 µm.

14. The fastening system of any of claims 1 to 13, wherein the closure elements are made of a monolayer film, e.g., a polyolefin film, a laminate of a nonwoven and a film layer, or a nonwoven without film layer.

15. The fastening system of claim 14, wherein the nonwoven is a spunbond nonwoven.

16. The fastening system of claim 15, wherein the film layer is extrusion-laminated to the spunbond nonwoven.

17. The fastening system of any of claims 1 to 16, wherein the pressure-sensitive adhesive is exposed on at least one closure element.

18. The fastening system of any of claims 1 to 17, wherein at least one of the closure elements is provided with a mechanical fastening element.

19. The fastening system of any of claims 1 to 18, further comprising a finger lift element on an outer edge of one of the closure elements.

20. The fastening system of any of claims 1 to 19, wherein said target tape is interposed between said fastening tab and said release tape.

21. The fastening system of claims 1 to 20, wherein the release tape is connected to the one of the closure elements by means of the target tape and to the other closure element by means of a center stripe.

22. The fastening system of any of claims 1 to 21, wherein the target tape comprises a finger lift element to enhance removability of the target tape from the release tape.

23. A method of manufacturing an elastic laminate fastening tab comprising the steps of:
i) providing a substantially inelastic web;
ii) coating one surface of the web with a pressure-sensitive adhesive;
iii) applying a blanking film onto the pressure-sensitive adhesive along the length of the web in a central portion of said web;
iv) slitting the web, the pressure-sensitive adhesive and the blanking film along their lengths;
v) attaching an elastic film to the two web portions on both sides of said blanking film to connect the two web portions, and
vi) cutting the thus formed laminate across its length to form said elastic laminate fastening tab.

24. A method of manufacturing a fastening system, particularly for adult incontinence diapers, said method comprising:
i) providing a substantially inelastic web;
ii) coating one surface of the web with a pressure-sensitive adhesive;
iii) applying a blanking film onto the pressure-sensitive adhesive along the length of the web in a central portion of said web;
iv) slitting the web, the pressure-sensitive adhesive and the blanking film along their lengths;
v) attaching an elastic film to the two web portions on both sides of said blanking film to connect the two web portions and to form a first laminate;
vi) providing a second laminate including a target tape, a release tape and a center stripe;
vii) laminating the first and second laminates together to provide a third laminate;
viii) rolling up said third laminate to form a laminate roll.

25. The method of claim 23 or 24, wherein the elastic film covers said blanking film completely and is directly attached to the two web portions.

26. The method of claim 23 or 24, wherein the blanking film is larger than the elastic film and the elastic film is attached to the two web portions via the blanking film.

27. The method of any of claims 23 to 26, wherein the elastic film is attached to the two web portions by means of adhesive bonding, thermobonding, ultrasonic bonding and/or cold pressure bonding.
